(19) Europäisches Patentamt
European Patent Office
Office européen des brevets

(11) EP 4 483 891 A1

(12) **EUROPEAN PATENT APPLICATION**
published in accordance with Art. 153(4) EPC

(43) Date of publication:
**01.01.2025 Bulletin 2025/01**

(21) Application number: **23806982.7**

(22) Date of filing: **17.05.2023**

(51) International Patent Classification (IPC):
***A61K 38/20*** *(2006.01)* ***A61K 47/60*** *(2017.01)*
***A61K 45/06*** *(2006.01)* ***A61P 35/00*** *(2006.01)*
***A61K 31/7068*** *(2006.01)*

(52) Cooperative Patent Classification (CPC):
(C-Sets available)
**A61K 47/60; A61K 31/7068; A61K 45/06** (Cont.)

(86) International application number:
**PCT/CN2023/094753**

(87) International publication number:
**WO 2023/222031 (23.11.2023 Gazette 2023/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.05.2022 CN 202210539593**

(71) Applicant: **Jenkem Technology Co., Ltd. (Beijing)**
**Haidian District**
**Beijing 100192 (CN)**

(72) Inventors:
- **LIU, Biao**
**Beijing 100192 (CN)**
- **WANG, Qingbin**
**Beijing 100192 (CN)**
- **ZHENG, Xichun**
**Beijing 100192 (CN)**
- **LI, Qiufen**
**Beijing 100192 (CN)**
- **WANG, Jie**
**Beijing 100192 (CN)**
- **GUO, Jun**
**Beijing 100192 (CN)**
- **ZHAO, Xuan**
**Beijing 100192 (CN)**

(74) Representative: **HGF**
**HGF Limited**
**1 City Walk**
**Leeds LS11 9DX (GB)**

# (54) PHARMACEUTICAL COMPOSITION FOR TREATING COLORECTAL CANCER AND USE THEREOF

(57) Provided are a pharmaceutical composition for treating colorectal cancer and use thereof. Active ingredients of the pharmaceutical composition comprise: polyethylene glycol modified IL-2 and capecitabine. The pharmaceutical composition shows a very good therapeutic effect in an animal model of colorectal cancer. The effect is remarkably superior to that of a single drug, and thus the pharmaceutical composition has a good application prospect.

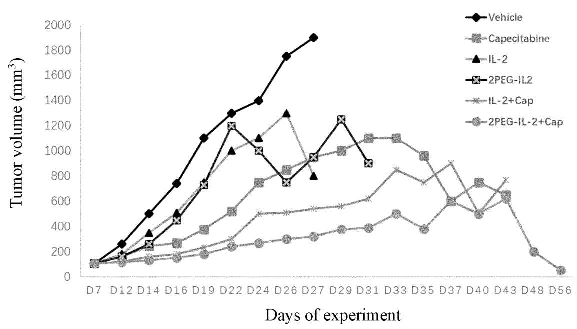


FIG. 6

EP 4 483 891 A1

(52) Cooperative Patent Classification (CPC): (Cont.)

C-Sets
**A61K 31/7068, A61K 2300/00**

## Description

TECHNICAL FIELD

The present invention relates to the technical field of chemical medicines, and particularly relates to a pharmaceutical composition for treating colorectal cancer and use thereof.

BACKGROUND

Colorectal cancer (CRC) is one of the most common malignancies of the digestive tract, with 1.1 million new cases and about 500,000 deaths worldwide each year. According to the latest global cancer burden data released by the World Health Organization's International Agency for Research on Cancer (IARC) in 2020, colorectal cancer has become the third most common cancer worldwide, following lung cancer. In recent years, with the aggravation of population aging and changes in dietary structure in China, the morbidity and mortality of colorectal cancer in China keep rising. The morbidity age also has a young trend, and most patients are already in the middle or late stages at the time of diagnosis. Therefore, reducing the morbidity and mortality of colorectal cancer in China is an urgent public health problem to be solved, and the development of new potent antitumor therapeutic drugs is still the main direction for current tumor therapy.

Interleukin 2 (IL-2) is a cytokine derived from multiple cells, having a pleiotropic effect, and it has an important effect on the immune response, virus infection resistance, etc., of organisms. IL-2 can activate T cells and stimulate the proliferation of T cells that are initiated by specific antigens, promoting the production of cytokines. It stimulates NK cell proliferation, enhances NK cell killing activity and cytokine generation, and induces the generation of LAK cells. Also, it promotes B cell proliferation and antibody secretion, and activates macrophages.

Since IL-2 can induce and enhance cytotoxic activity, research on the use of IL-2 for treating certain diseases, particularly for treating tumors, is widely carried out. Use of IL-2 alone or in combination with LAK cells and the like has achieved certain therapeutic effects in treating tumors, and IL-2 is also expected to be used in treating viral infections, immunodeficiency diseases, and autoimmune diseases. The recombinant IL-2 drug Proleukin (Aldesleukin), jointly developed by Novartis and linigen, was approved by the FDA for treating metastatic renal cancer in 1991, and it was approved for treating metastatic melanoma in 1998. However, due to its short half-life, narrow therapeutic window, significant toxic and side effects, and simultaneous stimulation of Treg activation, its clinical use is limited. In order to improve the pharmacokinetic properties and reduce toxic and side effects of IL-2, researchers change the affinity of IL-2 for different receptor subunits by means of amino acid mutation or chemical modification to increase activity and reduce toxicity. JK1219I is a biased agonist for CD122 (IL-2Rβ) antigen developed by Beijing Jenkem Technology Co., Ltd., and its design mechanism involves PEG modification of the α subunit of IL-2, so as to change the affinity of IL-2 for different receptor subunits, which stimulates the proliferation of tumor-killing T cells by targeting $CD8^+$ effector T cells, thereby utilizing a patient's own immune system to fight against cancer. The PEG modification not only changes the receptor selectivity of IL-2, but also greatly prolongs the half-life *in vivo,* effectively prolonging action duration and reducing toxic and side effects.

However, studies have found that the therapeutic effect of PEG-modified IL-2 on tumors such as colorectal cancer is still not ideal, and thus there is a need for further development of more effective drugs.

SUMMARY

In order to overcome the defects of the prior art, the present invention provides a pharmaceutical composition and use thereof, particularly use in the treatment of colorectal cancer.

In a first aspect of the present invention, provided is a pharmaceutical composition comprising polyethylene glycol-modified IL-2 and capecitabine as active ingredients. In one embodiment of the present invention, the active ingredients of the pharmaceutical composition consist of polyethylene glycol-modified IL-2 and capecitabine.

Specifically, in the pharmaceutical composition described above, the mass ratio of the polyethylene glycol-modified IL-2 to capecitabine may be in the range of 1:10 to 1:1000 (e.g., 1:10, 1:50, 1:100, 1:150, 1:200, 1:250, 1:300, 1:350, 1:400, 1:450, 1:500, 1:600, 1:700, 1:800, 1:900, or 1:1000), particularly in the range of 1: 100 to 1:500.

In another embodiment of the present invention, the active ingredients of the pharmaceutical composition further comprise other ingredients.

Specifically, the polyethylene glycol-modified IL-2 has the following structure:

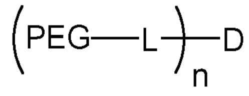

(I)

wherein PEG represents a polyethylene glycol residue;
L represents a linking group between PEG and D;
D represents a residue of interleukin 2; and
n is an integer of 1 to 12 (e.g., 1, 2, 3, 4, 5, 6, 7, 8, 9, 10, 11, or 12).

Specifically, n is an integer of 1 to 7; in some embodiments of the present invention, n is 1, 2, 3, or 4; particularly, n is 2.

Specifically, the interleukin 2 (IL-2) may be natural IL-2, recombinant IL-2, or a mutant having the same function as the natural IL-2; more specifically, the recombinant IL-2 may be a recombinant human interleukin 2 (rhIL-2).

Specifically, the linking site on IL-2 is an amino (or amine) group, such as one or more of the N-terminal amino group of the peptide chain, or a side-chain amino group of a lysine residue, a side-chain amino group of an arginine residue, a secondary amine group on imidazole ring of a histidine residue, and a secondary amine group on indole ring of a tryptophan residue in the peptide chain, particularly (one or more) side-chain amino group(s) of lysine residue(s) in the peptide chain.

In one embodiment of the present invention, L has the following structure: $-L_1-L_2-L_3$, wherein $L_1$ is selected from: a single bond, C1-6 alkylene, -(C1-6 alkylene)-$NR^{L1}$-, -(C1-6 alkylene)-O-, -(C1-6 alkylene)-S-, -(C1-6 alkylene)-$NR^{L1}$CO-, -(C1-6 alkylene)-CON$R^{L1}$-, -(C1-6 alkylene)-CO-, -(C1-6 alkylene)-OCO-, -(C1-6 alkylene)-NH-CON$R^{L1}$-, -(C1-6 alkylene)-$SO_2$-, and -(C1-6 alkylene)-SO-; $L_2$ is selected from: a single bond, C1-6 alkylene, cycloalkylene, arylene, and heterocyclylene; $L_3$ is selected from: C1-6 alkylene, -(C1-6 alkylene)-$NR^{L2}$-, -(C1-6 alkylene)-$NR^{L2}$-CO-, -(C1-6 alkylene)-CO-, and -(C1-6 alkylene)-OCO-; and $R^{L1}$ and $R^{L2}$ are independently selected from: H and C1-6 alkyl.

In some embodiments of the present invention, $R^{L1}$ and $R^{L2}$ are both H.

In some embodiments of the present invention, $L_3$ is -(C1-6 alkylene)-CO-.

In some embodiments of the present invention, $L_2$ is a single bond.

In some embodiments of the present invention, $L_1$ is selected from: a single bond, - (C1-6 alkylene)-NH-, -(C1-6 alkylene)-NHCO-, -(C1-6 alkylene)-CONH-, -(C1-6 alkylene)-CO-, and -(C1-6 alkylene)-OCO-.

In some embodiments of the present invention, L is -(C1-6 alkylene)-CO-, particularly $-CH_2CH_2-CO-$.

Specifically, the PEG may have a molecular weight of 300 to 100000 Daltons (specifically, e.g., 300, 500, 1000, 2000, 3000, 4000, 5000, 6000, 7000, 8000, 9000, 10000, 15000, 20000, 25000, 30000, 35000, 40000, 45000, 50000, 55000, 60000, 65000, 70000, 75000, 80000, 85000, 90000, 95000, and 100000), for example, 10000 to 60000 Daltons.

Specifically, the PEG may be a linear, Y-shaped, or multi-branched (e.g. four-, six-, or eight-arm) polyethylene glycol residue.

In one embodiment of the present invention, the PEG has the following structure:

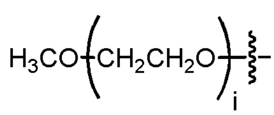

(II)

wherein i is an integer of 5 to 2500 (specifically, e.g., 5, 10, 20, 30, 40, 50, 100, 200, 300, 400, 500, 600, 700, 800, 900, 1000, 1100, 1200, 1300, 1400, 1500, 1600, 1700, 1800, 1900, 2000, 2100, 2200, and 2500).

In another embodiment of the present invention, the PEG has the following structure:

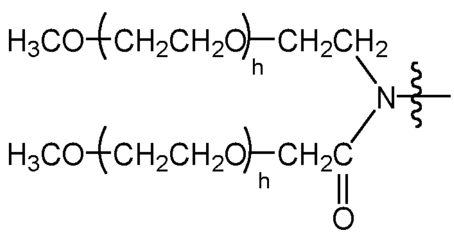

(III),

or

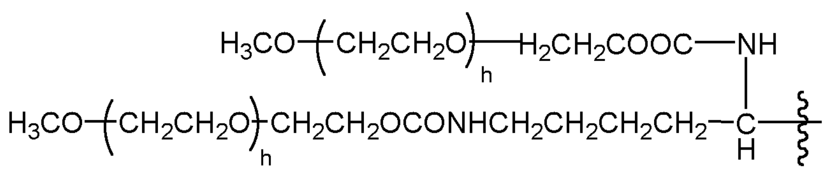

(IV)

wherein h is an integer of 3 to 1200 (specifically, e.g., 3, 5, 10, 20, 30, 40, 50, 100, 150, 200, 250, 300, 350, 400, 450, 500, 550, 600, 650, 700, 800, 900, 1000, 1100, and 1200).

In another embodiment of the present invention, the PEG described above has the following structure:

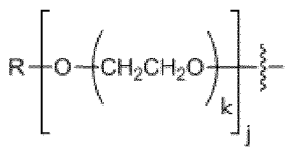

(V)

wherein:

k is an integer of 1 to 800 (specifically, e.g., 1, 5, 10, 20, 30, 40, 50, 100, 150, 200, 250, 300, 350, 400, 450, 500, 550, 600, 650, 700, 750, and 800);

j is an integer of 3 to 12 (e.g., 3, 4, 5, 6, 7, 8, 9, 10, 11, or 12); and

R is a residue of a core molecule of a multi-branched polyethylene glycol selected from pentaerythritol, methyl glucoside, sucrose, diethylene glycol, propylene glycol, glycerol, or polyglycerol.

In some embodiments of the present invention, the PEG has the following structure:

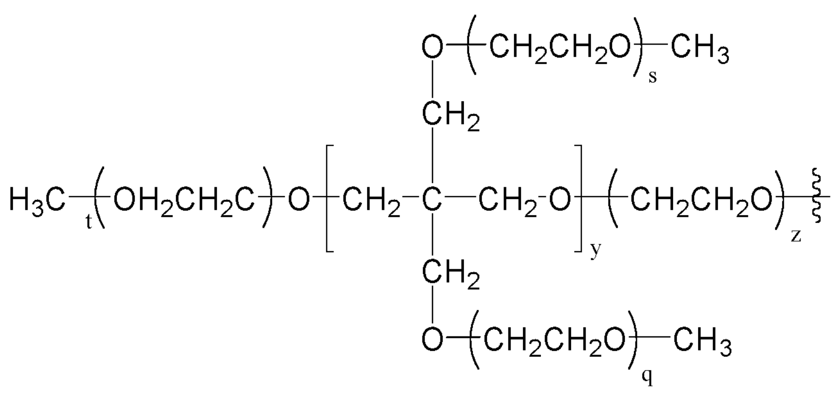

(VI)

wherein q, s, t, and z are independently selected from an integer of 2 to 340, and y is an integer of 1 to 5 (e.g., 1, 2, 3, 4, or 5).

In other embodiments of the present invention, the PEG has the following structure:

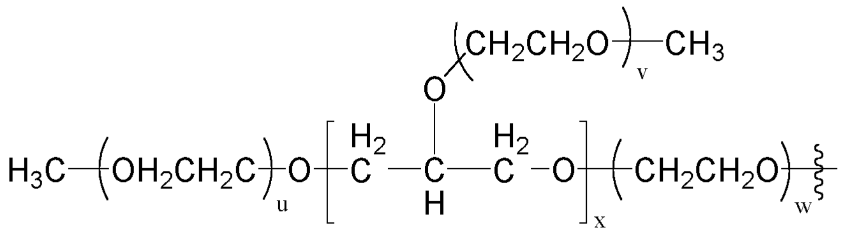

(VII)

wherein u, v, and w are independently selected from an integer of 2 to 460, and x is an integer of 1 to 5 (e.g., 1, 2, 3, 4, or 5).

In some embodiments of the present invention, the polyethylene glycol-modified IL-2 has the following structure:

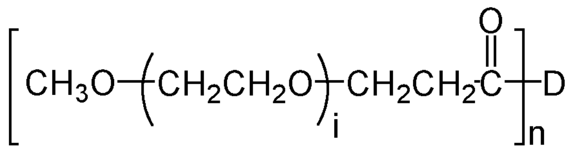

(VIII)

wherein n may be 1, 2, 3, or 4; particularly, n is 2.

Specifically, the polyethylene glycol residue in the polyethylene glycol-modified IL-2 as shown in formula VIII may have a molecular weight of 10000 to 60000 Daltons, such as 20000 Daltons.

Specifically, the polyethylene glycol-modified IL-2 described above can be prepared (and separated) by the reaction of a polyethylene glycol modifier with an IL-2 protein (particularly, a certain molar ratio of the polyethylene glycol modifier to the IL-2 protein), for example, reaction with shaking in a pH neutral buffer at room temperature.

In some embodiments of the present invention, the polyethylene glycol modifier is

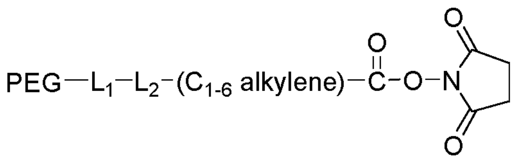

,

for example

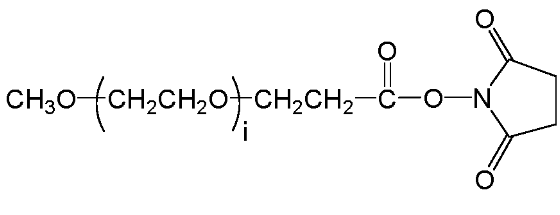

,

and its molecular weight may be 300 to 100000 Daltons, or 10000 to 60000 Daltons, such as 20000 Daltons.

Specifically, in the pharmaceutical composition, the two active ingredients described above may be administered by the same or different administration routes. For example, polyethylene glycol-modified IL-2 may be administered by a parenteral route (e.g., administered intravenously, intramuscularly, intradermally, subcutaneously or intraperitoneally), while capecitabine is administered by a gastrointestinal route (e.g., administered orally).

Specifically, in the pharmaceutical composition, the two active ingredients described above may be formulated for simultaneous, separate, or sequential administration. Specifically, the pharmaceutical composition further comprises one or more pharmaceutically acceptable auxiliary materials.

Specifically, the pharmaceutically acceptable auxiliary materials refer to conventional pharmaceutical auxiliary

materials in the pharmaceutical field, such as diluents, fillers, wetting agents, binders, disintegrants, lubricants, flavoring agents, solubilizers, cosolvents, antioxidants, pH regulators, isotonic regulators, bacteriostats, solvents, and the like.

In some embodiments of the present invention, the pharmaceutical composition further comprises oral dosage form auxiliary materials (such as diluents, fillers, wetting agents, binders, disintegrants, lubricants, flavoring agents, antioxidants, bacteriostats, etc.), and injection auxiliary materials (such as solubilizers, cosolvents, antioxidants, pH regulators, isotonic regulators, bacteriostats, solvents, etc.).

Specifically, the pharmaceutical composition described above may be in an oral dosage form, such as a tablet (for example, but not limited to, a tablet, a pill, a powder, a granule, a capsule, a troche, a syrup, a liquid, an emulsion, a suspension, etc.), an injection (for example, subcutaneous injection, intravenous injection, intramuscular injection, and intraperitoneal injection), and the like.

In some embodiments of the present invention, the pharmaceutical composition comprises an injection dosage form comprising the polyethylene glycol-modified IL-2 as an active ingredient, and an oral dosage form comprising capecitabine as an active ingredient.

In a second aspect of the present invention, provided is use of a combination of a polyethylene glycol-modified IL-2 and capecitabine in preparing a medicament for treating a tumor.

Specifically, the polyethylene glycol-modified IL-2 is as described in the first aspect of the present invention.

Specifically, in the use, the tumor is a primary tumor or metastatic tumor.

Specifically, in the use, the tumor is rectal cancer, colon cancer, colorectal cancer, breast cancer, large intestine cancer, or gastric cancer, particularly advanced rectal cancer, colon cancer, colorectal cancer, breast cancer, large intestine cancer, or gastric cancer.

In some preferred embodiments of the present invention, in the use, the tumor is advanced rectal cancer, colon cancer, or colorectal cancer.

The present invention also provides use of the pharmaceutical composition described in the first aspect in preparing a medicament for enhancing the efficacy of polyethylene glycol-modified IL-2 or capecitabine, and reducing the side effect of polyethylene glycol-modified IL-2 or capecitabine.

The present invention also provides use of a polyethylene glycol-modified IL-2 in preparing a medicament for enhancing the efficacy of capecitabine, and reducing the side effect of capecitabine.

The present invention also provides use of capecitabine in preparing a medicament for enhancing the efficacy of polyethylene glycol-modified IL-2, and reducing the side effect of polyethylene glycol-modified IL-2.

Specifically, in the uses described above, the polyethylene glycol-modified IL-2 is as described in the first aspect of the present invention.

Specifically, in the uses described above, the efficacy is an antitumor effect, such as inhibiting tumor growth (e.g., reducing the tumor volume), and prolonging the survival time of subjects.

Specifically, in the uses described above, the side effect is pulmonary edema, capillary leakage, and the like.

Specifically, in the uses described above, the tumor may be rectal cancer, colon cancer, colorectal cancer, breast cancer, large intestine cancer, or gastric cancer, particularly advanced rectal cancer, colon cancer, colorectal cancer, breast cancer, large intestine cancer, or gastric cancer.

In a third aspect of the present invention, provided is a method for treating a tumor, comprising a step of administering to a subject in need thereof a polyethylene glycol-modified IL-2 in combination with capecitabine, or the pharmaceutical composition described in the first aspect of the present invention.

Specifically, in the method, the tumor is rectal cancer, colon cancer, colorectal cancer, breast cancer, large intestine cancer, or gastric cancer, particularly advanced rectal cancer, colon cancer, colorectal cancer, breast cancer, large intestine cancer, or gastric cancer.

In some preferred embodiments of the present invention, in the method, the tumor is advanced rectal cancer, colon cancer, or colorectal cancer.

Specifically, the polyethylene glycol-modified IL-2 is as described in the first aspect of the present invention.

In one embodiment of the present invention, the method comprises a step of administering the polyethylene glycol-modified IL-2 by a parenteral route (e.g., intravenous injection).

In one embodiment of the present invention, the method comprises a step of administering capecitabine by a gastrointestinal route (e.g., oral administration). Specifically, the subject may be a mammal, such as a human, monkey, pig, cow, horse, sheep, dog, cat, mouse, etc., particularly a human.

The present invention provides a brand-new pharmaceutical composition showing a very good therapeutic effect on an animal model of colon cancer, which is remarkably superior to that of a single drug. The pharmaceutical composition is intended to provide an effective treatment mode for patients with advanced primary or metastatic breast cancer, rectal cancer, colon cancer, gastric cancer, and the like.

BRIEF DESCRIPTION OF THE DRAWINGS

FIG. 1 shows the HPLC detection spectrum of PEGylation reaction products.
FIG. 2 shows the CM chromatography separation spectrum of PEGylation reaction products.
FIG. 3 shows the purity analysis spectrum of 2PEG-IL2 sample.
FIG. 4 shows the fitted curve of a standard.
FIG. 5 shows the fitted curve of a test sample.
FIG. 6 is a graph showing the changes in tumor volume of mice in all groups.

DETAILED DESCRIPTION

Unless otherwise defined, all scientific and technical terms used in the present invention have the same meaning as commonly understood by those skilled in the art to which the present invention relates.

The term "alkyl" refers to a hydrocarbon chain radical that is linear or branched and that does not contain unsaturated bonds, and that is linked to the rest of the molecule via a single bond. Typical alkyl groups contain 1 to 12 (e.g., 1, 2, 3, 4, 5, 6, 7, 8, 9, 10, 11, or 12) carbon atoms, preferably 1 to 6 carbon atoms; examples include methyl, ethyl, *n*-propyl, isopropyl, *n*-butyl, isobutyl, *tert*-butyl, *n*-pentyl, isopentyl, neopentyl, *tert*-pentyl, *n*-hexyl, isohexyl, etc.

The term "alkylene" refers to hydrocarbyl (divalent alkyl) formed from an alkane molecule by losing two hydrogen atoms, which may be linear or branched and is linked to other parts of the molecule by a single bond. Typical alkylene contains 1 to 12 (e.g., 1, 2, 3, 4, 5, 6, 7, 8, 9, 10, 11, or 12) carbon atoms, preferably 1 to 6 carbon atoms; examples include methylene ($-CH_2-$), ethylidene ($-CH_2CH_2-$), propylene ($-CH_2CH_2CH_2-$, $-CH(CH_3)CH_2-$, $-CH_2CH(CH_3)-$), butylene, and the like.

The disclosures of the various publications, patents, and published patent specifications cited herein are hereby incorporated by reference in their entireties.

The technical solutions of the present invention will be clearly and completely described below with reference to the examples of the present invention, and it is obvious that the described examples are only a part of the examples of the present invention but not all of them. Based on the examples of the present invention, all other examples obtained by those of ordinary skills in the art without creative work shall fall within the protection scope of the present invention.

Example 1: Preparation of PEGylation Reaction Mixture

IL-2 protein (provided by Beijing Jenkem Technology Co., Ltd.) was taken and exchanged into a 5 mM PB buffer at pH 7.0, and a PEG modifier (provided by Beijing Jenkem Technology Co., Ltd.) was added according to a certain reaction molar ratio. The mixture was mixed well and reacted at room temperature with shaking for 2 h. After the reaction was finished, the preparation condition of the reaction mixture was detected by an HPLC method. The results are shown in FIG. 1 and Table 1.

The structure of the used PEG modifier is shown as follows, with a molecular weight being 20,000 Daltons:

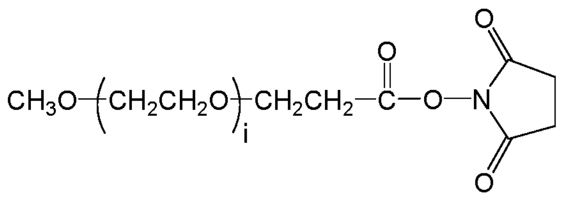

Table 1. Composition analysis of reaction mixture (UV-0217-0002)

| Peak number | Peak name | Retention time | Peak height | Peak area | Content |
|---|---|---|---|---|---|
| 1 | 4PEG-IL2 | 28.457 | 7663.378 | 259121.828 | 1.8749 |
| 2 | 3PEG-IL2 | 29.333 | 43000.484 | 1744956.750 | 12.6260 |
| 3 | 2PEG-IL2 | 30.470 | 121325.367 | 5250551.500 | 37.9913 |
| 4 | 1PEG-IL2 | 32.112 | 118941.758 | 4828735.000 | 34.9392 |
| 5 | IL-2 | 34.543 | 86735.430 | 1737025.500 | 12.5686 |

The results show that the proportion of 2PEG-IL2 was about 37%.

Example 2: Separation of 2PEG-IL2 by CM Chromatography

The reaction mixture obtained in Example 1 was diluted with water while the pH was adjusted to 3.0 and the conductivity to 1.5 mS/cm, and loaded onto an equilibrated CM column by AKTA chromatography system. 10 mM sodium acetate buffer at pH 3.0 served as phase A, and 10 mM sodium acetate buffer at pH 3.0 containing 1 M NaCl served as phase B. The elution was carried out using a gradient elution method with phase B, and the eluted fractions were collected when the concentration of phase B reached 8%. The purity of the collected fractions was analyzed by HPLC, yielding a 2PEG-IL2 sample.

The separated 2PEG-IL2 sample was exchanged into 5 mM PB buffer at pH 7.0 using a G25 desalting column for later use.

The CM chromatography separation spectrum is shown in FIG. 2. Using a CM ion exchange column chromatography resin, the samples conjugated to different numbers of PEGs were separated by a gradient elution mode with increasing salt concentration. The peaks, from left to right, correspond to 3PEG-IL2, 2PEG-IL2, 1PEG-IL2, and IL-2. The purity of the samples in different collection tubes was determined in combination with an HPLC detection method. The 2PEG-IL2 sample was mainly centered on the left second peak.

Example 3: Alpha Affinity Chromatography

The specific procedures are as follows:

(1) a filler was equilibrated with 5 mM PB buffer at pH 7.0;
(2) the sample obtained in Example 2 was loaded into the affinity filler;
(3) elution was performed with a prepared eluent, and the elution sample was collected;
(4) the collected elution sample was exchanged into a 5 mM PB buffer at pH 7.0 by using a G25 desalting column;
(5) the sample obtained in step (4) was concentrated, and filtered and sterilized through a 0.22 μm filter membrane. The purity was detected by HPLC.

The purity analysis spectrum of the 2PEG-IL2 sample is shown in FIG. 3, indicating that the finally required 2PEG-IL2 is obtained.

Example 4: Evaluation of 2PEG-IL2's Impact on Mouse T Lymphocyte Proliferation *In Vitro*

1. Cell suspension preparation:
10-30 mL of CTLL-2 cell culture was taken and placed into a 50 mL sterile centrifuge tube, and centrifuged at 2000 r/min for 7 min to collect the cells. The supernatant was discarded, and the cells were washed twice with phosphate buffered saline (DPBS), which was added to about 25 mL each time, followed by centrifugation at 2000 r/min for 7 min. After one more wash with 25 mL of 1640 culture medium (centrifuged at 2000 r/min for 7 min), the cells were resuspended in a basic culture medium, and counted using a cell counting plate. The cell concentration was adjusted to about $6.0 \times 10^5$ cells/mL for later use.
2. Pre-dilution of the standard and test sample:

(1) The standard (IL-2 protein) was diluted with a basic culture medium to 200 IU/mL according to its potency.
(2) The stock solution of the test sample (2PEG-IL2 purified in Example 3) was diluted with a basic culture medium to 1300 IU/mL according to specific activity of $1.7\times10^7$.

3. Plating and sample loading:
50 μL/well of basic culture medium was pre-added to a 96-well cell culture plate. The standard solution and test sample solution were subsequently subjected to a 2-fold serial dilution, with 8 dilutions made for each, leaving 50 μL in each well, and 2 replicate wells were made for each gradient.
4. Addition of cell suspension:
50 μL/well of the prepared cell suspension was added to the 96-well plate from low concentration to high concentration. The cell suspension was pipetted with a multichannel pipette until the cells were fully and uniformly mixed, and then added in the air.
5. Culturing:

The plate was incubated at a condition of 37 ± 1°C and 5 ± 1% $CO_2$ for 18-24 h.
6. Staining:
20 μL/well of 5 mg/mL MTT solution was added, and the plate was incubated at a condition of 37 ± 1°C and 5 ± 1% $CO_2$ for 4-6 h.
7. Lysis:
150 μL/well of 15% SDS lysis solution was added, and the plate was incubated at a condition of 37 ± 1°C and 5 ± 1% $CO_2$ for 18-24 h.
8. Plate reading:
After vibration and mixing uniformly, a color comparison was carried out on a microplate reader, with an experimental wavelength of 570 nm and a reference wavelength of 630 nm.

The results are shown in Tables 2-4.

Table 2. Absorbance at the wavelength of 570 nm

| Dilution factor | Standard | | | Test sample | | |
|---|---|---|---|---|---|---|
| | OD value | | CV(%) | OD value | | CV(%) |
| 1 | 0.6702 | 0.6885 | 1.905 | 0.4842 | 0.4990 | 2.129 |
| 2 | 0.6592 | 0.6745 | 1.622 | 0.4145 | 0.4169 | 0.4082 |
| 4 | 0.6445 | 0.6734 | 3.101 | 0.3530 | 0.3638 | 2.131 |
| 6 | 0.5853 | 0.6337 | 5.615 | 0.2934 | 0.2936 | 0.04818 |
| 16 | 0.5018 | 0.5307 | 3.958 | 0.2562 | 0.2553 | 0.2488 |
| 32 | 0.3636 | 0.3411 | 4.515 | 0.2384 | 0.2472 | 2.563 |
| 64 | 0.3093 | 0.3456 | 7.839 | 0.2251 | 0.2451 | 6.015 |
| 128 | 0.3023 | 0.2655 | 9.166 | 0.2314 | 0.2218 | 2.996 |

Table 3. Absorbance at the wavelength of 630 nm

| Dilution factor | Standard | | | Test sample | | |
|---|---|---|---|---|---|---|
| | OD value | | CV(%) | OD value | | CV(%) |
| 1 | 0.3178 | 0.3293 | 2.513 | 0.2376 | 0.2464 | 2.571 |
| 2 | 0.2939 | 0.3239 | 6.867 | 0.2074 | 0.2118 | 1.484 |
| 4 | 0.3022 | 0.3172 | 3.425 | 0.1791 | 0.1832 | 1.600 |
| 6 | 0.2753 | 0.2900 | 3.678 | 0.1541 | 0.1509 | 1.484 |
| 16 | 0.2413 | 0.2524 | 3.180 | 0.1373 | 0.1370 | 0.1547 |
| 32 | 0.1815 | 0.1728 | 3.473 | 0.1274 | 0.1325 | 2.775 |
| 64 | 0.1642 | 0.1988 | 13.48 | 0.1215 | 0.1388 | 9.399 |
| 128 | 0.1763 | 0.1443 | 14.12 | 0.1237 | 0.1195 | 2.442 |

Table 4. Absorbance difference at wavelength of 570 nm-630 nm

| Dilution factor | OD difference of standard | | OD difference of test sample | |
|---|---|---|---|---|
| 1 | 0.3524 | 0.3592 | 0.2466 | 0.2526 |
| 2 | 0.3653 | 0.3506 | 0.2071 | 0.2051 |
| 4 | 0.3423 | 0.3562 | 0.1739 | 0.1806 |
| 6 | 0.3100 | 0.3437 | 0.1393 | 0.1427 |
| 16 | 0.2605 | 0.2783 | 0.1189 | 0.1183 |
| 32 | 0.1821 | 0.1683 | 0.1110 | 0.1147 |

(continued)

| Dilution factor | OD difference of standard | | OD difference of test sample | |
|---|---|---|---|---|
| 64 | 0.1451 | 0.1468 | 0.1036 | 0.1063 |
| 128 | 0.1260 | 0.1212 | 0.1077 | 0.1023 |

According to the results in Table 4, the fitted curve of the standard is obtained as shown in FIG. 4, and y = 0.356578 + ((0.124514 - 0.356578)/(1 + (x/19.3893)^-2.28678)); $R^2$ = 0.998.

According to the results in Table 4, the fitted curve of the test sample is obtained as shown in FIG. 5, and y = 0.312763 + ((0.100927 - 0.312763)/(1 + (x/2.13405)^-1.08894)); $R^2$ = 0.997.

$$\text{Biological activity of test sample (IU/mL)} = \text{biological activity of standard} \times \frac{C1}{C2} \times \frac{D1}{D2} = 1.2 \times 10^5\ \text{IU/mL}$$

wherein:

$C_1$ is the dilution factor of the test sample equivalent to the median effective dose of the standard;
$C_2$ is the dilution factor of the median effective dose of the standard;
$D_1$ is the pre-dilution factor of the test sample;
$D_2$ is the pre-dilution factor of the standard;

$$\text{Specific activity of the test sample (IU/mg)} = \frac{\text{Protein content of test sample (mg/mL)}}{\text{Biological activity of test sample(IU/mL)}} = 1.6 \times 10^5\ \text{IU/mg}$$

The results show that 2PEG-IL2 can significantly stimulate CTLL-2 cell proliferation, and that the isolated 2PEG-IL2 prepared in Examples 1-3 has good biological activity, but its biological activity is slightly lower compared to IL-2.

Example 5: Effect of Combined Administration of 2PEG-IL2 and Capecitabine (Cap) on CT26 Cell Mouse Transplantation Tumor Model

1. CT26 cells were cultured in an RPMI-1640 culture medium to the logarithmic growth phase, digested with pancreatin, centrifuged, and adjusted to a cell concentration of 5 × $10^6$/mL with PBS buffer.
2. 100 Balb/c mice were inoculated with 0.1 mL of tumor cell fluid subcutaneously on the right back of each mouse, and the mice were bred in an SPF clean room. When the tumors grew to 100-150 $mm^3$, the animals were randomly divided into 6 groups, with 10 in each group. Specific groups and modes of administration are given in the following table:

Table 5 Experimental groups and modes of administration

| **Group** | | **Dose (mg/kg)** | **Administration volume (mL/kg)** | **Administration mode, frequency, and period** | **Number of animals** |
|---|---|---|---|---|---|
| 1 | Vehicle control group | - | - | IV, QD*5, 2weeks | 10 |
| 2 | Capecitabine | 500 | 5 | PO, QD*5, 2weeks | 10 |
| 3 | IL-2 | 3.0 | 5 | IV, QD*5, 2weeks | 10 |
| 4 | 2PEG-IL2 | 2.0 | 5 | IV, single-dose/week, 2weeks | 10 |
| 5 | IL-2 +Cap | IL-2 (3.0mg/kg) + Cap (500mg/kg) | 5 | IV, QD*5, 2weeks+ PO, QD*5, 2weeks | 10 |

(continued)

| Group | | Dose (mg/kg) | Administration volume (mL/kg) | Administration mode, frequency, and period | Number of animals |
|---|---|---|---|---|---|
| 6 | 2PEG-IL2 +Cap | 2PEG-IL2 (2.0mg/kg+ Cap (500mg/kg) | 5 | IV, single-dose/week, 2weeks + PO, QD*5, 2weeks | 10 |

3. The growth of the tumors was dynamically observed and weighed during the experiment, and the long diameter (a) and the short diameter (b) of the tumors were measured by a vernier caliper. The tumor-bearing mice were killed at the experimental end point, and the tumor bodies were stripped. The stripped tumors were cleaned of bloodstains, fat, and other non-tumor tissues, and the tumor tissues were separately preserved in liquid nitrogen and 4% paraformaldehyde. Tumor volume and tumor volume inhibition rate were calculated according to the formula: mean tumor volume ($mm^3$) = $ab^2/2$; tumor volume inhibition rate (%) = (1 - mean tumor volume in experimental group/mean tumor volume in negative control check) $\times$ 100%.

4. Experimental results

The tumor volume changes and survival curves of the mice in each group are shown in FIG. 6.

Analysis from the aspect of efficacy: (1) During the experiment, the tumors in the mice of the solvent control check showed a gradual upward trend, indicating that the mouse model of colon cancer was successfully established and could be used for efficacy evaluation. (2) The antitumor curative effect of the combination of IL-2 and capecitabine is significantly superior to that of a single drug. (3) The efficacy of the combination of 2PEG-IL2 and capecitabine is significantly better than that of the combined administration of IL-2 and capecitabine. The reason may be that PEG modification of the $\alpha$ subunit of IL-2 changes the affinity of IL-2 for different receptor subunits, which stimulates the proliferation of tumor-killing T cells by targeting $CD8^+$ effector T cells, while reducing the immunosuppression caused by Treg, thereby utilizing the mice's own immune system to fight against the tumor. The results of the survival experiment are consistent with the tumor volume results, suggesting that the combination of 2PEG-IL2 and capecitabine has good antitumor effects on the mouse model of colon cancer.

Analysis from the aspect of safety: The mice in the group treated with the combination of IL-2 and capecitabine all died around 42 days after administration. Pulmonary edema in the mice was discovered through dissection, and the pathological section result showed leakage in the pulmonary capillaries. This phenomenon is consistent with the side effects of IL-2 reported in the literature. However, the mean survival time of mice in the group treated with the combination of 2PEG-IL2 and capecitabine was significantly longer than that of the group treated with the combination of IL-2

and capecitabine. At the experimental end point, phenomena of pulmonary edema, capillary leakage, and the like were not found in the autopsied mice. The reason may be that PEG modification greatly prolongs the half-life of IL-2 *in vivo,* effectively extending the action duration, reducing the administration dose, and decreasing the administration frequency, so that the toxic and side effects were significantly lower than those of IL-2.

In conclusion: The combination of 2PEG-IL2 and capecitabine has good antitumor effects on the mouse model of colon cancer, and both its efficacy and safety are significantly superior to those of the combination of IL-2 and capecitabine. In addition, the reduced administration dose and administration frequency are conducive to improving patient compliance, which is helpful for the clinical promotion and application.

The above description is only for the purpose of illustrating the preferred examples of the present invention, and is not intended to limit the scope of the present invention. Any modifications, equivalents, and the like made without departing from the spirit and principle of the present invention shall fall in the protection scope of the present invention.

The foregoing examples and methods described herein may vary based on the abilities, experience, and preferences of those skilled in the art.

The certain order in which the steps of the method are listed in the present invention does not constitute any limitation on the order of the steps of the method.

## Claims

**1.** A pharmaceutical composition, comprising or consisting of the following as active ingredients: polyethylene glycol-modified IL-2 and capecitabine,

preferably, the polyethylene glycol-modified IL-2 has the following structure:

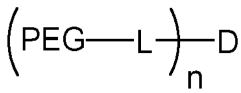

(I)

wherein PEG represents a polyethylene glycol residue;
L represents a linking group between PEG and D;
D represents a residue of interleukin 2; and
n is an integer of 1 to 12.

2. The pharmaceutical composition according to claim 1, wherein n is an integer of 1 to 7, preferably 2.

3. The pharmaceutical composition according to claim 1, wherein L has the following structure:

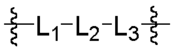

, wherein $L_1$ is selected from a single bond, C1-6 alkylene, - (C1-6 alkylene)-$NR^{L1}$-, -(C1-6 alkylene)-O-, -(C1-6 alkylene)-S-, -(C1-6 alkylene)-$NR^{L1}CO$-, -(C1-6 alkylene)-$CONR^{L1}$-, -(C1-6 alkylene)-CO-, -(C1-6 alkylene)-OCO-, -(C1-6 alkylene)-$NHCONR^{L1}$-, -(C1-6 alkylene)-$SO_2$-, and -(C1-6 alkylene)-SO-; $L_2$ is selected from a single bond, C1-6 alkylene, cycloalkylene, arylene, and heterocyclylene; $L_3$ is selected from C1-6 alkylene, -(C1-6 alkylene)-$NR^{L2}$-, -(C1-6 alkylene)-$NR^{L2}CO$-, -(C1-6 alkylene)-CO-, and -(C1-6 alkylene)-OCO-; and $R^{L1}$ and $R^{L2}$ are independently selected from H and C1-6 alkyl;

preferably, $L_3$ is -(C1-6 alkylene)-CO-;
more preferably, L is -(C1-6 alkylene)-CO-.

4. The pharmaceutical composition according to claim 1, wherein the interleukin 2 is natural IL-2, recombinant IL-2, or a mutant having the same function as the natural IL-2; preferably, the recombinant IL-2 is a recombinant human interleukin 2.

5. The pharmaceutical composition according to any one of claims 1-4, wherein the PEG has a molecular weight of 300 to 100000 Daltons, preferably of 10000 to 60000 Daltons;

preferably, the PEG has the following structure:

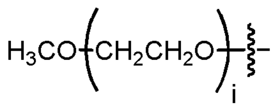

(II)

wherein i is an integer of 5 to 2500; or,
the PEG has the following structure:

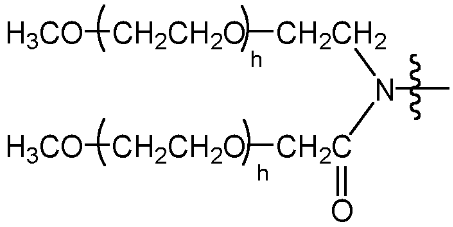

(III),

or

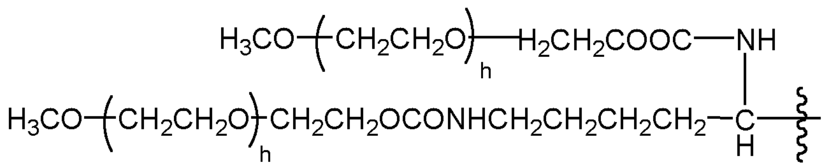

(IV)

wherein h is an integer of 3 to 1200; or,
the PEG has the following structure:

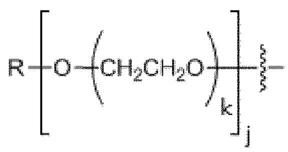

(V)

wherein:

k is an integer of 1 to 800;
j is an integer of 3 to 12;
R is a residue of a core molecule of a multi-branched polyethylene glycol selected from pentaerythritol, methyl glucoside, sucrose, diethylene glycol, propylene glycol, glycerol, or polyglycerol;
more preferably, the PEG has the following structure:

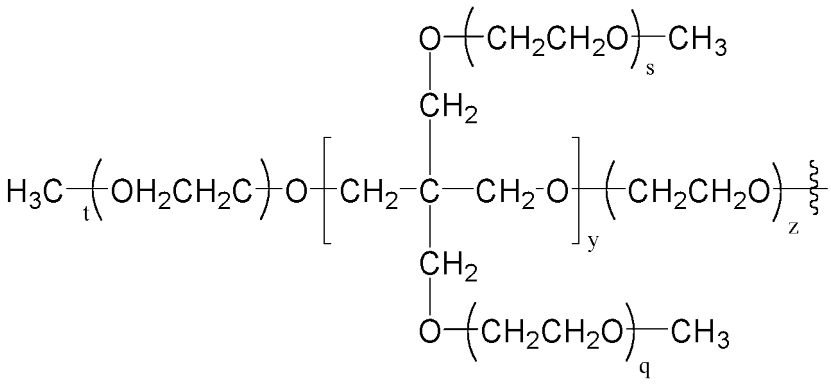

(VI)

wherein q, s, t, and z are independently selected from an integer of 2 to 340, y is an integer of 1 to 5; or,
the PEG has the following structure:

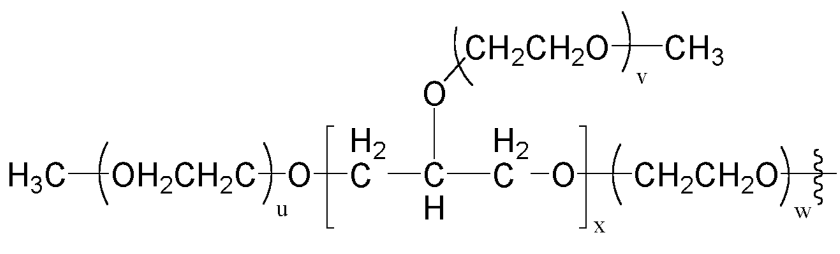

(VII)

wherein u, v, and w are independently selected from an integer of 2 to 460, and x is an integer of 1 to 5.

6. The pharmaceutical composition according to claim 5, wherein the polyethylene glycol-modified IL-2 has the following structure:

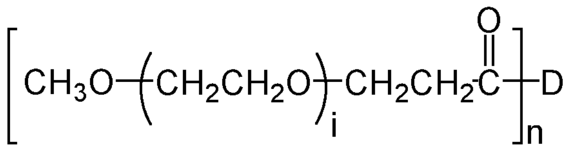

(VIII)

preferably, n is 1, 2, 3, or 4.

7. The pharmaceutical composition according to claim 1, wherein the pharmaceutical composition comprises an injection dosage form comprising the polyethylene glycol-modified IL-2 as an active ingredient, and an oral dosage form comprising capecitabine as an active ingredient.

8. Use of a combination of a polyethylene glycol-modified IL-2 and capecitabine in preparing a medicament for treating a tumor,

preferably, the polyethylene glycol-modified IL-2 has the following structure:

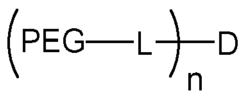

(I)

wherein PEG represents a polyethylene glycol residue;
L represents a linking group between PEG and D;
D represents a residue of interleukin 2; and
n is an integer of 1 to 12.

9. The use according to claim 8, wherein n is an integer of 1 to 7, preferably 2.

10. The use according to claim 8, wherein L has the following structure: $-L_1-L_2-L_3-$, wherein $L_1$ is selected from a single bond, C1-6 alkylene, -(C1-6 alkylene)-$NR^{L1}$-, - (C1-6 alkylene)-O-, -(C1-6 alkylene)-S-, -(C1-6 alkylene)-$NR^{L1}$-CO-, -(C1-6 alkylene)-$CONR^{L1}$-, -(C1-6 alkylene)-CO-, -(C1-6 alkylene)-OCO-, -(C1-6 alkylene)-$NHCONR^{L1}$-, -(C1-6 alkylene)-$SO_2$-, and -(C1-6 alkylene)-SO-; $L_2$ is selected from a single bond, C1-6 alkylene, cycloalkylene, arylene, and heterocyclylene; $L_3$ is selected from C1-6 alkylene, -(C1-6 alkylene)-$NR^{L2}$-, -(C1-6 alkylene)-$NR^{L2}CO$-, -(C1-6 alkylene)-CO-, and -(C1-6 alkylene)-OCO-; and $R^{L1}$ and $R^{L2}$ are independently selected from H and C1-6 alkyl;

preferably, $L_3$ is -(C1-6 alkylene)-CO-;
more preferably, L is -(C1-6 alkylene)-CO-.

11. The use according to claim 8, wherein the interleukin 2 is natural IL-2, recombinant IL-2, or a mutant having the same function as the natural IL-2; preferably, the recombinant IL-2 is a recombinant human interleukin 2.

12. The use according to any one of claims 8-11, wherein the PEG has a molecular weight of 300 to 100000 Daltons, preferably of 10000 to 60000 Daltons;

preferably, the PEG has the following structure:

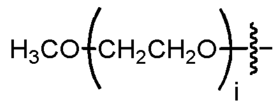

(II)

wherein i is an integer of 5 to 2500; or,
the PEG has the following structure:

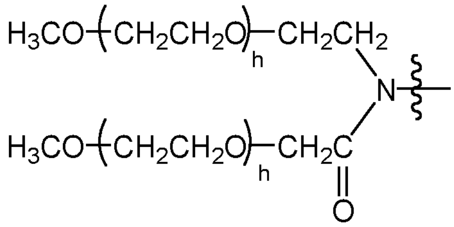

(III),

or

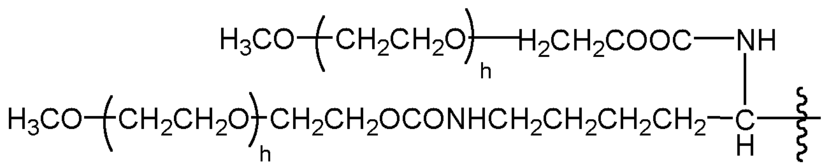

(IV)

wherein h is an integer of 3 to 1200; or,
the PEG has the following structure:

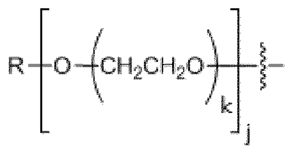

(V)

wherein:

k is an integer of 1 to 800;
j is an integer of 3 to 12;
R is a residue of a core molecule of a multi-branched polyethylene glycol selected from pentaerythritol, methyl glucoside, sucrose, diethylene glycol, propylene glycol, glycerol, or polyglycerol;
more preferably, the PEG has the following structure:

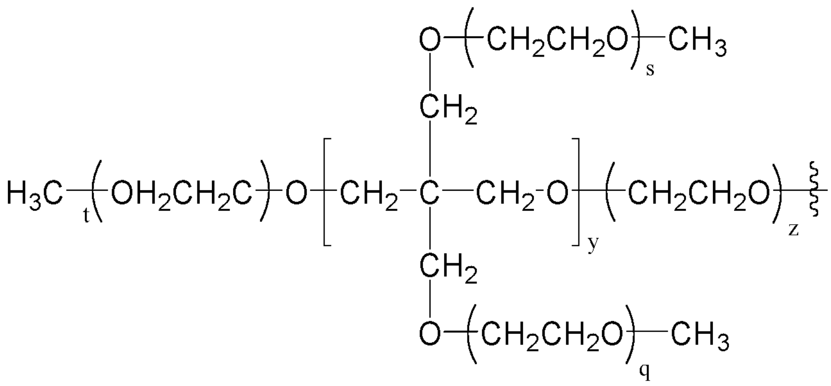

(VI)

wherein q, s, t, and z are independently selected from an integer of 2 to 340, y is an integer of 1 to 5; or, the PEG has the following structure:

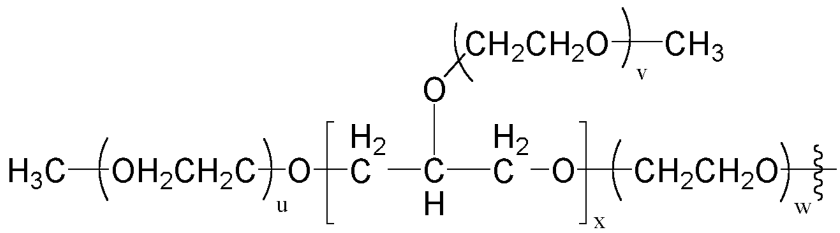

(VII)

wherein u, v, and w are independently selected from an integer of 2 to 460, and x is an integer of 1 to 5.

**13.** The use according to claim 12, wherein the polyethylene glycol-modified IL-2 has the following structure:

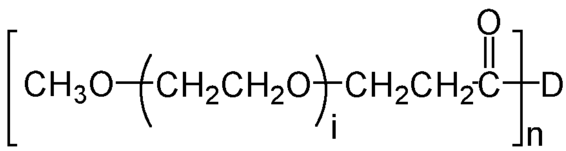

(VIII)

preferably, n is 1, 2, 3, or 4.

**14.** The use according to claim 8, wherein the tumor is rectal cancer, colon cancer, colorectal cancer, breast cancer, large intestine cancer, or gastric cancer, preferably advanced rectal cancer, colon cancer, colorectal cancer, breast cancer, large intestine cancer, or gastric cancer.

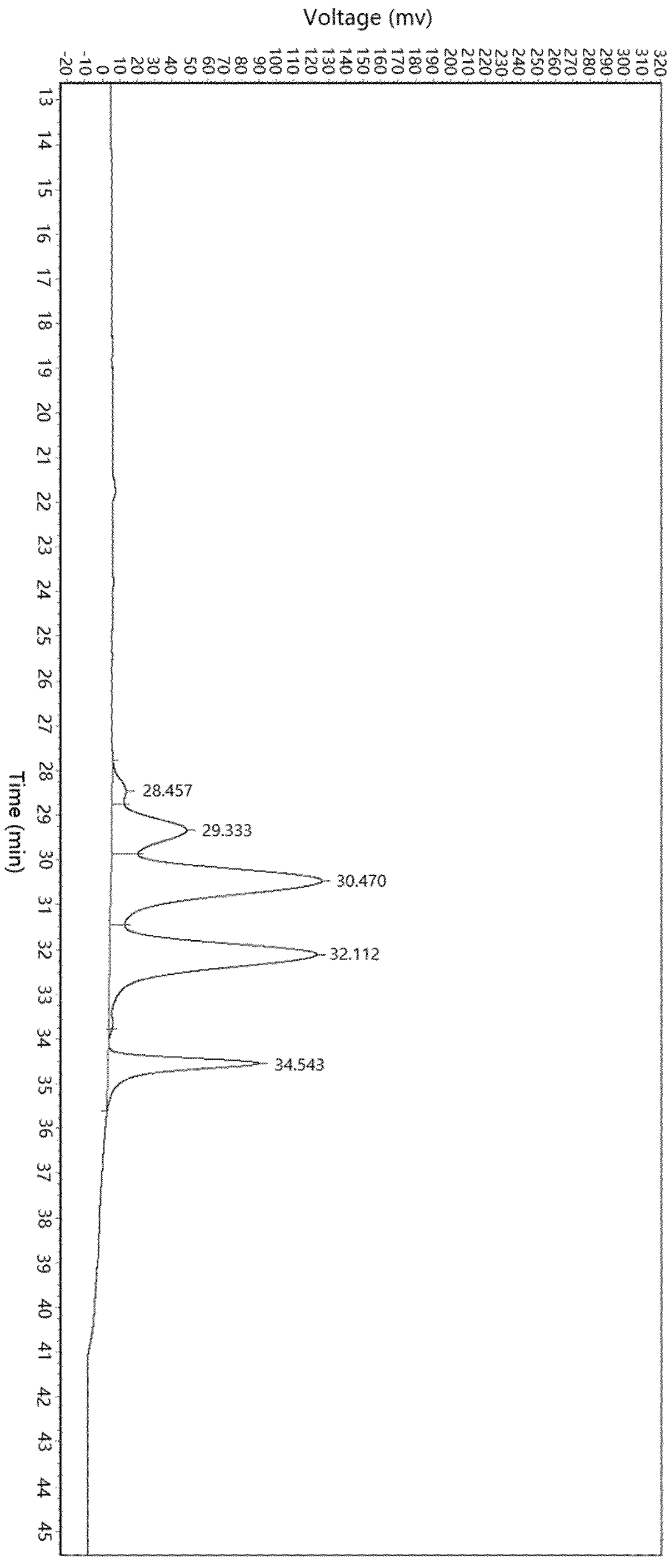


FIG. 1

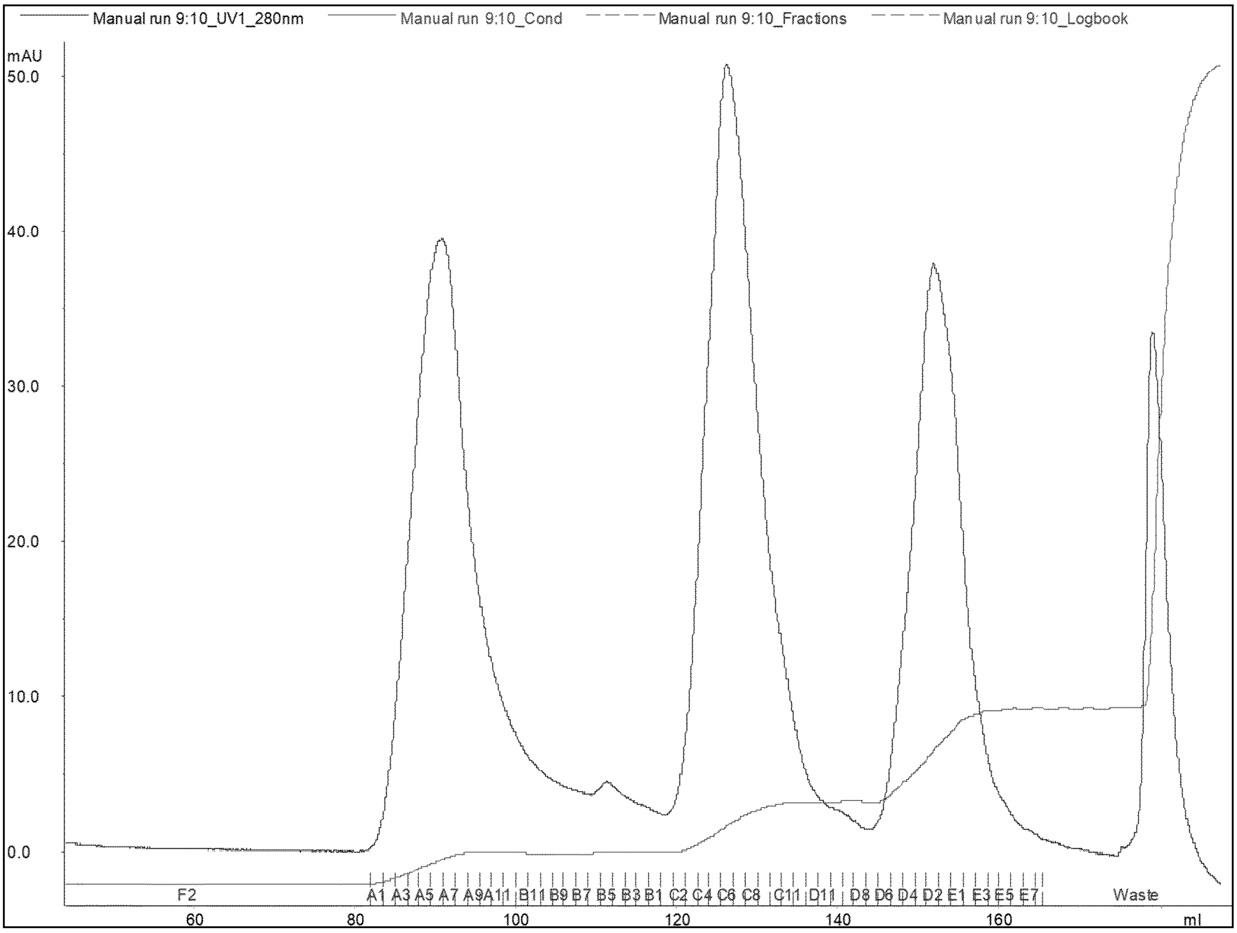
Manual run 9:10_UV1_280nm
Manual run 9:10_Cond
Manual run 9:10_Fractions
Manual run 9:10_Logbook
mAU
50.0
40.0
30.0
20.0
10.0
0.0
F2
A1 A3 A5 A7 A9A11 B11 B9 B7 B5 B3 B1 C2 C4 C6 C8 C11 D11 D8 D6 D4 D2 E1 E3 E5 E7
Waste
60
80
100
120
140
160
ml

FIG. 2

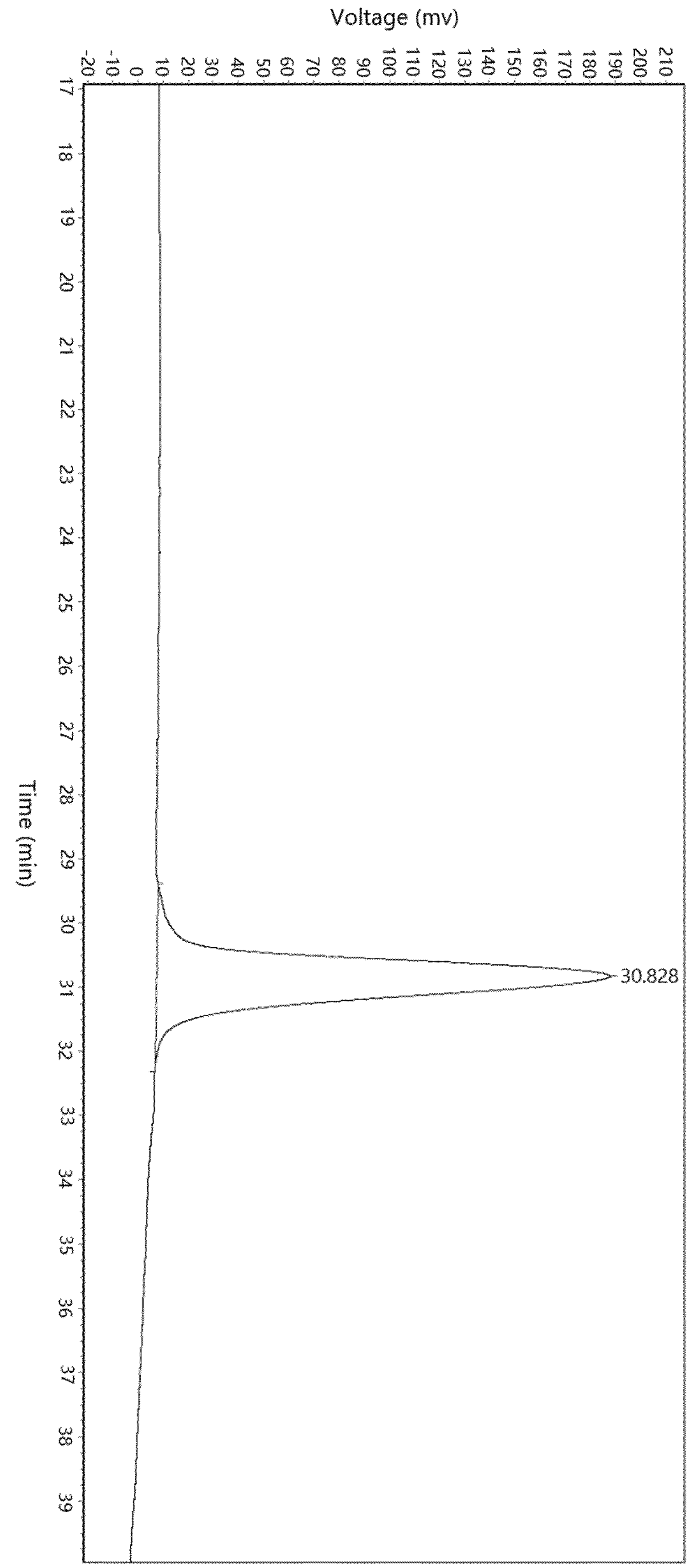


FIG. 3

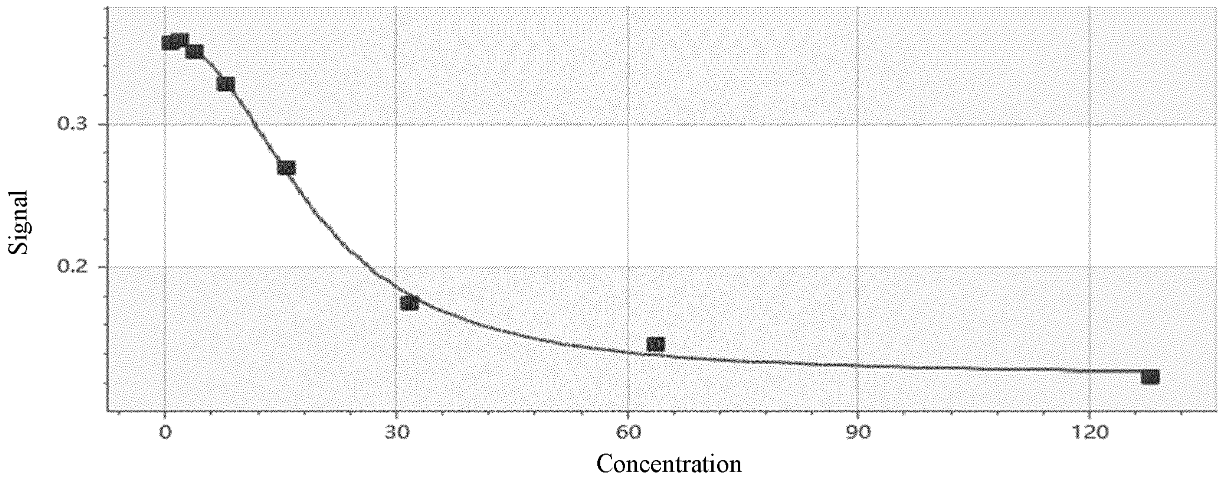
Signal
0.3
0.2
0
30
60
90
120
Concentration

FIG. 4

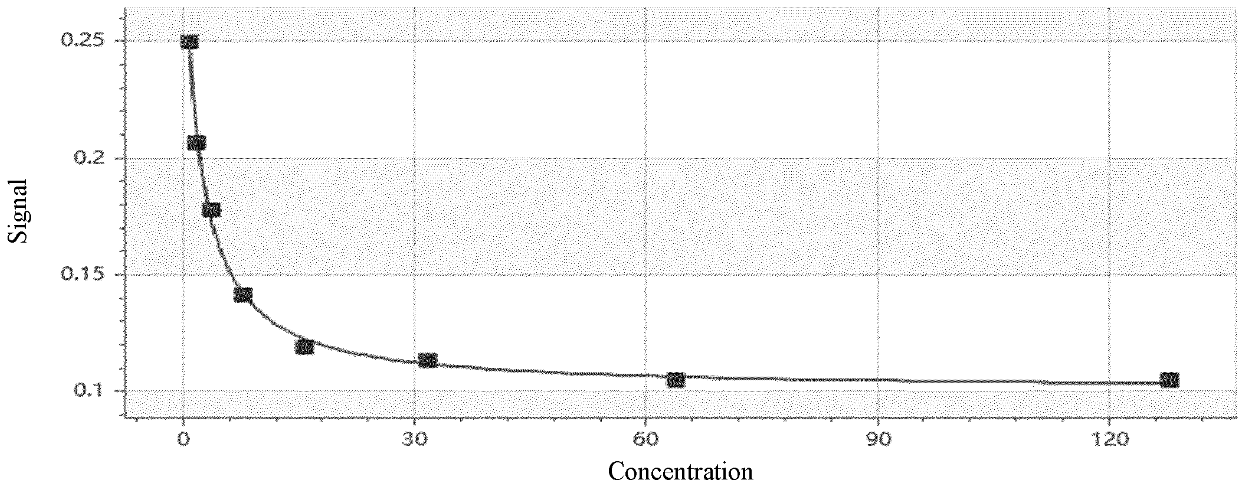
Signal
0.25
0.2
0.15
0.1
0
30
60
90
120
Concentration

FIG. 5

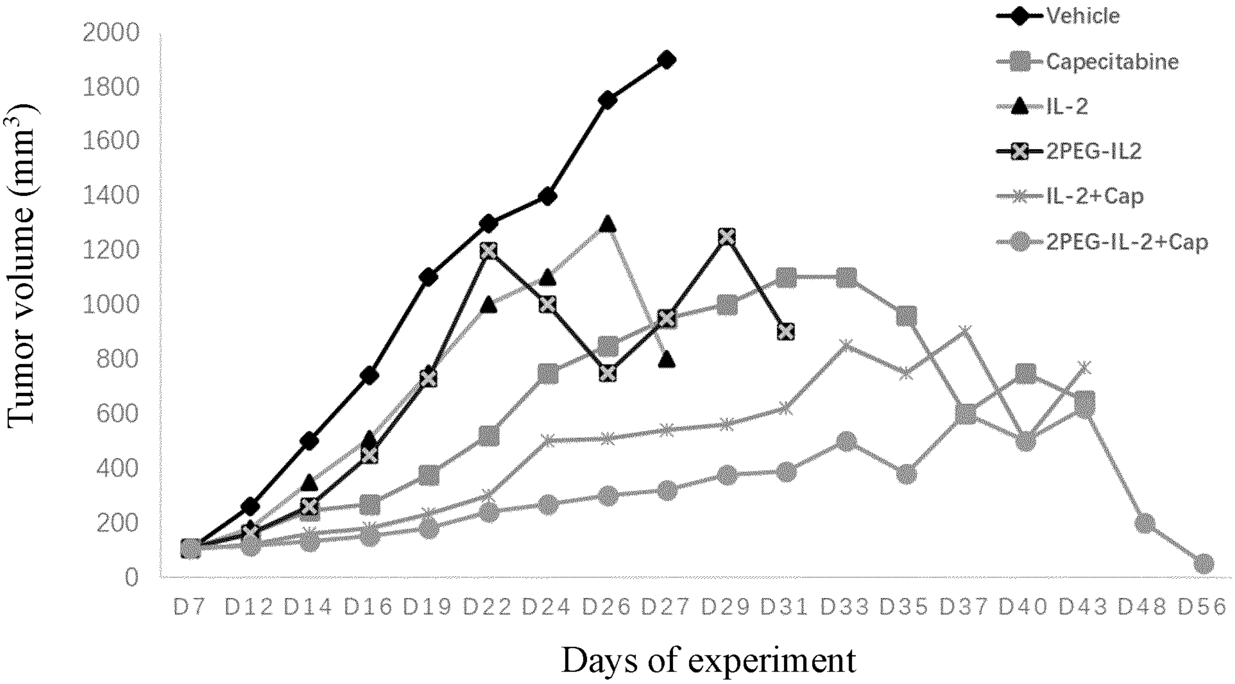
Tumor volume (mm3)
2000
1800
1600
1400
1200
1000
800
600
400
200
0
D7 D12 D14 D16 D19 D22 D24 D26 D27 D29 D31 D33 D35 D37 D40 D43 D48 D56
Days of experiment
Vehicle
Capecitabine
IL-2
2PEG-IL2
IL-2+Cap
2PEG-IL-2+Cap

FIG. 6

## INTERNATIONAL SEARCH REPORT

International application No.
**PCT/CN2023/094753**

**A. CLASSIFICATION OF SUBJECT MATTER**

A61K38/20(2006.01)i; A61K47/60(2017.01)i; A61K45/06(2006.01)i; A61P35/00(2006.01)i; A61K31/7068(2006.01)i

According to International Patent Classification (IPC) or to both national classification and IPC

**B. FIELDS SEARCHED**

Minimum documentation searched (classification system followed by classification symbols)

IPC: A61K,A61P

Documentation searched other than minimum documentation to the extent that such documents are included in the fields searched

Electronic data base consulted during the international search (name of data base and, where practicable, search terms used)

CNABS, CNTXT, VEN, ENTXT, ENTXTC, VCN, CNKI, 万方, WANFANG, PUBMED, ISI WEB OF KNOWLEDGE, BING: IL-2, 白细胞介素-2, 白介素-2, interleukin-2, 卡培他滨, capecitabine, 联合, combination, 聚乙二醇, PEGylated, 肿瘤, 癌, 结肠癌, 直肠癌, 结直肠癌, 大肠癌, tumor, cancer, colon, colorectal, 键凯科技股份有限公司, 刘彪, 王庆彬, 刘喜春, 李秋芬, 王杰, 郭军, 赵宣

**C. DOCUMENTS CONSIDERED TO BE RELEVANT**

| Category* | Citation of document, with indication, where appropriate, of the relevant passages | Relevant to claim No. |
|---|---|---|
| PX | CN 114767835 A (BEIJING JENKEM TECHNOLOGY CO., LTD.) 22 July 2022 (2022-07-22)<br>claims 1-14 | 1-14 |
| Y | CN 113121670 A (TIANJIN JENKEM TECHNOLOGY CO., LTD.) 16 July 2021 (2021-07-16)<br>description, paragraphs 8-26, 47-56, and 61, and embodiments 5-7 | 1-14 |
| Y | 余培东 等 (YU, Peidong et al.). "结肠癌术后卡培他滨联合白细胞介素-2化疗对免疫功能的影响 (Non-official translation: Effect of Postoperative Capecitabine Combined with Interleukin-2 Chemotherapy on Immune Function After Colon Cancer Surgery)" *山西医药杂志 (Shanxi Medical Journal),* Vol. 36, No. 10, 31 October 2007 (2007-10-31),<br>page 918 | 1-14 |
| Y | CN 109589415 A (TIANJIN JENKEM TECHNOLOGY CO., LTD.) 09 April 2019 (2019-04-09)<br>description, paragraphs 5-87, 160, 228-240, and 253, and embodiment 14 | 1-14 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 August 2023** | **17 August 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/094753**

**C. DOCUMENTS CONSIDERED TO BE RELEVANT**

| Category* | Citation of document, with indication, where appropriate, of the relevant passages | Relevant to claim No. |
|---|---|---|
| Y | CHARYCH, D.H. et al. "NKTR-214, an Engineered Cytokine with Biased IL2 Receptor Binding, Increased Tumor Exposure, and Marked Efficacy in Mouse Tumor Models" *Clin Cancer Res,* Vol. vol. 22, No. 3, 01 February 2016 (2016-02-01), abstract, and page 682, right column, paragraph 4 to page 684, right column, paragraph 1 | 1-14 |
| Y | 黄丽晶 等 (HUANG, Lijing et al.). "IL-2修饰药物分子研发进展 (Advances of Modified IL-2 Molecules in Drug Development)" *生物工程学报 (Chinese Journal of Biotechnology),* Vol. 38, No. 9, 31 March 2022 (2022-03-31), page 3282, left column, paragraph 1 to page 3283, left column, paragraph 1 | 1-14 |
| Y | 郭建峰 等 (GUO, Jianfeng et al.). "卡培他滨治疗晚期转移性结直肠癌的疗效观察 (Efficacy of Capecitabine in Patients with Metastatic Colorectal Cancer)" *慢性病学杂志 (Chronic Pathematology Journal),* Vol. 12, No. 3, 31 March 2010 (2010-03-31), page 208, left column, paragraph 2 to right column, paragraph 3 | 1-14 |
| Y | 马玉彦 等 (MA, Yuyan et al.). "5-Fu与IL-2联合应用对体外培养癌细胞的抑制率的影响 (Non-official translation: Effect of the Combination of 5-Fu and IL-2 on the Inhibition Rate of Cancer Cells Cultured in Vitro)" *实用肿瘤学杂志 (Journal of Practical Oncology),* Vol. 16, No. 4, 15 November 2002 (2002-11-15), abstract, and page 260, right column, paragraph 3 to page 261, left column, paragraph 1 | 1-14 |
| A | LI, J.J. et al. "The Role of Interleukins in Colorectal Cancer" *Int. J. Biol. Sci.,* Vol. 16, 14 June 2020 (2020-06-14), pages 2323-2339 | 1-14 |
| A | 陈强 等 (CHEN, Qiang et al.). "白介素-2在结直肠癌生物治疗中的应用 (Non-official translation: Application of Interleukin-2 in the Biological Therapy of Colorectal Cancer)" *外科理论与实践 (Journal of Surgery Concepts & Practice),* Vol. 11, No. 1, 31 December 2006 (2006-12-31), pages 69-71 | 1-14 |

Form PCT/ISA/210 (second sheet) (July 2022)

**INTERNATIONAL SEARCH REPORT**
**Information on patent family members**

International application No.
**PCT/CN2023/094753**

| Patent document cited in search report | | | Publication date (day/month/year) | Patent family member(s) | | | Publication date (day/month/year) |
|---|---|---|---|---|---|---|---|
| CN | 114767835 | A | 22 July 2022 | None | | | |
| CN | 113121670 | A | 16 July 2021 | None | | | |
| CN | 109589415 | A | 09 April 2019 | US | 2020289655 | A1 | 17 September 2020 |

Form PCT/ISA/210 (patent family annex) (July 2022)